# EUROPÄISCHE PATENTSCHRIFT

(11) **EP 1 558 592 B1**
(45) Veröffentlichungstag und Bekanntmachung des Hinweises auf die Patenterteilung: **17.02.2010**
(21) Anmeldenummer: 03753557.2
(22) Anmeldetag: 15.10.2003
(51) Int. Cl.: C07D 277/56, A01N 43/78

(54) **THIAZOL-(BI)CYCLOALKYL-CARBOXANILIDE**
THIAZOL-(BI)CYCLOALKYL-CARBOXANILIDES
THIAZOL-(BI)CYCLOALKYLCARBOXANILIDES

(30) Priorität: 28.10.2002 DE 10250110
(43) Veröffentlichungstag der Anmeldung: 03.08.2005
(73) Patentinhaber: Bayer CropScience Aktiengesellschaft, 40789 Monheim (DE)
(72) Erfinder: DUNKEL, Ralf, 69001 Lyon (DE); RIECK, Heiko, F-69110 Ste. Foy-lès-Lyon (FR); ELBE, Hans-Ludwig, 42329 Wuppertal (DE); GREUL, Jörg, Nico, 42799 Leichlingen (DE); WACHENDORFF-NEUMANN, Ulrike, 56566 Neuwied (DE); KUCK, Karl-Heinz, 40764 Langenfeld (DE); DAHMEN, Peter, 41470 Neuss (DE)
(86) Internationale Anmeldenummer: PCT/EP2003/011392
(87) Internationale Veröffentlichungsnummer: WO 2004/039789

(56) Entgegenhaltungen:
- EP-A- 0 276 177
- EP-A- 0 315 502
- EP-A- 0 371 950
- EP-A- 0 591 699
- WO-A-02/08197
- WO-A-02/059086
- WO-A-03/066610
- WO-A-03/074491

## Beschreibung

Die vorliegende Erfindung betrifft neue Thiazol-(bi)cyclalkyl-carboxanilide, mehrere Verfahren zu deren Herstellung und deren Verwendung zur Bekämpfung von unerwünschten Mikroorganismen.

Es ist bereits bekannt, dass zahlreiche Thiazol-(bi)cycloalkyl-carboxanilide fungizide Eigenschaften besitzen (vgl. z.B. WO 02/059086, EP-A 0 591 699, EP-A 0 589 301, EP-A 0 545 099, EP-A 0 315 502 und EP-A 0 276 177). So offenbart WO-A 2004/039 verschiedene mit einer Oximgruppe substituierte Biphenylcarboxamide, die an der Carbonylfunktion eine Thiazolylgruppe enthalten können. Aus EP-A 0371950 sind Trifluoro-methyl-Thiazolylphenylcarboxamide bekannt, die am Phenylrest kleine aliphatische Substituenten wie zum Beispiel Halogen, Halogenalkyl, Halogenalkoxy enthalten. WO-A 2003/074491 zeigt o-Cyclopropylphenyl-Carboxamide, die an der Carbonylfunktion eine Trifluoromethyl substituierte Thiazolylgruppe enthalten können und zusätzlich am Cyclopropyl substituiert sind. Schlussendlich werden in der WO-A 2003/0666610 Difluoromethyl-Thiazolyl-Biphenylcarboxamide offenbart, die bis zu fünffach am zweiten Phenylrest der Biphenylgruppe substituiert sein können.
So sind z.B. bereits die folgenden Thiazol-(bi)cycloalkyl-eaeboxanilide bekannt geworden: 2-Amino-*N*-(2-cyclohexylphenyl)4-(trifluormethyl)-1,3-thiazol-5-carboxamid und 2-Amino-*N*-(2-cyclopentylphenyl-4-(trifluormethyl)-1,3-thiazol-5-carboxamid aus EP-A 0 589 301, *N*-(2-Cyclohexylphenyl)-2-methyl-4-(trifluormethyl)-1,3-thiazol-5-carboxamid und *N*-(2-Cyclopentylphenyl)-2-methyl-4-(tnifluonnethyl)-1,3-thiazol-5-carboxamid aus EP-A 0 545 099, 2-Methyl-4-(trifluonnethyl)-*N*-(1,1,3-trimethyl-2,3-dihydro-1*H-*inden-4-yl)-1,3-thiazol-5-carboxamid aus EP-A 0 276 177 und 2-Methyl-4-(trifluonnethyl)-*N-*(1,1,3-trimethyl-1,3-dihydro-2-benzofuran-4-yl)-1,3-thiazol-5-carboxamid aus EP-A 0 315 502. Die Wirksamkeit dieser Stoffe ist gut, lässt aber in manchen Fällen, z.B. bei niedrigen Aufwandmengen zu wünschen übrig.

Es wurden nun neue Thiazol-(bl)cycloalkyl-earboxanilide der Formel (I) 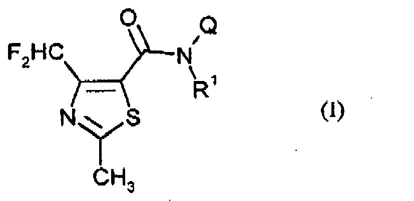 gefunden, in welcher
- Q: für eine Gruppe 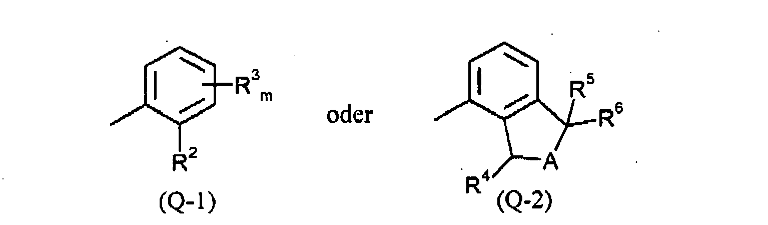 steht,
- R¹: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylsulfanyl, C₁-C₄-Halogenalkylsulfinyl, C₁-C₄-Halogenalkylsulfonyl, Halogen-C₁-C₄-atkoxy-C₁-C₄-alkyl, C₃-C₈-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
- R²: für jeweils gegebenenfalls einfach oder mehrfach, gleich oder verschieden durch Halogen, Cyano, Hydroxy, C₁-C₈-Alkyl, C₁-C₈-Alkoxy, C₁-C₆-Halogenalkyl, C₁-C₆-Halogenalkoxy mit jeweils 1 bis 9 Fluor-, Chlor- und/oder Bromatomen substituiertes C₃-C₁₂-Cycloalkyl, C₃-C₁₂-Cycloalkenyl, C₆-C₁₂-Bicycloalkyl oder C₆-C₁₂-Bicycloalkenyl steht,
- R³: für Fluor, Chlor, Brom oder Methyl steht,
- m: für 0, 1, 2, 3 oder 4 steht,
- A: für O (Sauerstoff) oder CR¹² steht,
- R⁴, R⁵, R⁶ und R¹²: unabhängig voneinander für Wasserstoff, Methyl oder Ethyl stehen,
- R⁷: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen oder 4-(Difluormethyl)-2-methyl-1,3-thiazol-2-yl steht,
- R⁸ und R⁹: unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
- R⁸ und R⁹: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
- R¹⁰ und R¹¹: unabhängig voneinander für Wasserstoff, C₁-C₈-Alkyl, C₃-C₈-Cyaoalkyl; C₁-C₈-Halogenalkyl, C₃-C₈-Halogencycloalkyl mit jeweils 1 bis 9 Fluor-, Chlor- und/oder Bromatomen stehen,
- R¹⁰ und R¹¹: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
- R¹³: für Wasserstoff oder C₁-C₆-Alkyl steht.

Weiterhin wurde gefunden, dass man Thiazol-(bi)cycloalkyl-carboxanilide der Formel (I) erhält, indem man
A) Carbonsäure-Derivate der Formel (II) 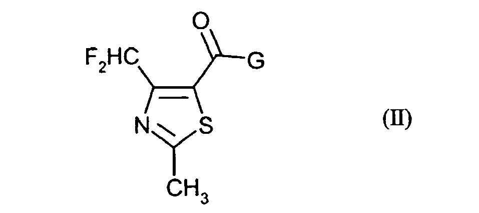 in welcher
   - G: für Halogen, Hydroxy oder C₁-C₆-Alkoxy steht,
   in einem ersten Schritt mit Anilin-Derivaten der Formel (III)

   H₂N-Q (III)

   in welcher
   - Q: die oben angegebenen Bedeutungen hat,
   in Gegenwart eines Säurebindemittels und in Gegenwart eines Verdünnungsmittels umsetzt
   und die so erhaltenen Produkte der Formel (I-a) 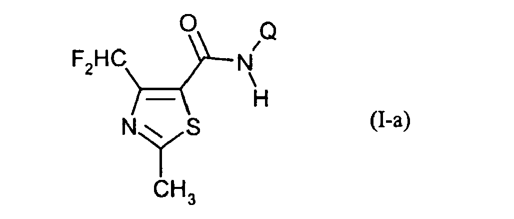 in welcher
   - Q: die oben angegebenen Bedeutungen hat,
   gegebenenfalls in einem zweiten Schritt mit einem Halogenid der Formel (III)

   R¹⁻¹-X (IV)

   in welcher
   - R¹⁻¹: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylsulfanyl, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
   - R⁷, R⁸, R⁹, R¹⁰ und R¹¹: die oben angegebenen Bedeutungen haben und
   - X: für Chlor, Brom oder Iod steht,
   in Gegenwart einer Base und in Gegenwart eines Verdünnungsmittels umsetzt.

Schließlich wurde gefunden, dass die neuen Thiazol-(bi)cycloalkyl-carboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Thiazol-(bi)cycloalkyl-carboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Thiazol-(bi)cycloalkyl-carboxanilide sind durch die Formel (I) allgemein definiert. Bevorzugte Substituenten bzw. Bereiche der in den oben und nachstehend erwähnten Formeln aufgeführten Reste werden im Folgenden erläutert.
- R¹: steht bevorzugt für Wasserstoff; C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylsulfanyl, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen; -COR⁷, -CONR⁸R⁹ oder -CH₂NR¹⁰R¹¹.
- R¹: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylsulfanyl, Difluorchlormethylsulfanyl, Trifluormethylsulfanyl, Trifluormethylsulfinyl, Trifluormethylsulfonyl, Trifluormethoxymethyl; -COR⁷, -CONR⁸R⁹ oder -CH₂NR¹⁰R¹¹.
- R¹: steht ganz besonders bevorzugt für Wasserstoff; Methyl, Methoxymethyl oderCOR⁷.
- R²: steht bevorzugt für jeweils gegebenenfalls einfach bis vierfach, gleich oder verschieden durch Fluor, Chlor, Brom, Cyano, Hydroxy, C₁-C₆-Alkyl, C₁-C₆-Alkoxy, C₁-C₄-Halogenalkyl, C₁-C₄-Halogenalkoxy mit jeweils 1 bis 9 Fluor-, Chlor- und/oder Bromatomen substituiertes C₃-C₁₂-Cycloalkyl substituiertes C₃-C₁₂-Cyclaoalkyl, C₃-C₁₂-Cycloalkenyl, C₆-C₁₂-Bicycloalkyl oder C₆-C₁₂-Bicycloalkenyl.
- R²: steht besonders bevorzugt für jeweils gegebenenfalls einfach bis dreifach, gleich oder verschieden durch Fluor, Chlor, Brom, Cyano, Hydroxy, Methyl, Ethyl, n- oder iso-Propyl, n-, iso-, sec- oder tert-Butyl, Methoxy, Ethoxy, n- oder iso-Propoxy, n-, iso-, sec- oder tert-Butoxy, Trifluormethyl, Difluormethyl, Trichlormethyl, Difluorchlormethyl, Trifluormethoxy, Difluormethoxy, Trichlormethoxy, Difluorchlormethoxy substituiertes C₃-C₁₀-Cycloalkyl, C₃-C₁₀-Cycloalkenyl, C₆-C₁₀-Bicycloalkyl oder C₆-C₁₀-Bicycloalkenyl.
- R²: steht ganz besonders bevorzugt für jeweils einfach bis dreifach, gleich oder verschieden durch Fluor, Chlor, Brom, Methyl, Ethyl, iso-Propyl, tert-Butyl, Methoxy, Ethoxy, iso-Propoxy, tert-Butoxy, Trifluormethyl, Trifluormethoxy substituiertes Cyclopropyl, Cyclopentyl, Cyclohexyl, Cyclopentyl, Cyclohexyl, Cycloheptyl, Cyclooctyl, Cyclohexenyl, Cycloheptenyl, Cyclooctenyl, Bicyclo[4.1.0]heptyl, Bicyclo[2.2.1]heptyl.
- R³: steht bevorzugt für Fluor, Brom oder Methyl.
- R³: steht besonders bevorzugt für Fluor oder Methyl.
- m: steht bevorzugt für 0, 1, 2 oder 3.
- m: steht besonders bevorzugt für 0, 1 oder 2.
- A: steht bevorzugt für O (Sauerstoff).
- A: steht bevorzugt für CR¹².
- R⁴: steht bevorzugt für Methyl oder Ethyl.
- R⁴: steht besonders bevorzugt für Methyl.
- R⁵ und R⁶: stehen bevorzugt jeweils für Methyl.
- R⁷: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen oder 4-(Difluormethyl)-2-methyl-1,3-thiazol-2-yl.
- R⁷: steht besonders bevorzugt für Wasserstoff, Methyl, Ethyl, n- oder iso-Propyl, tert-Butyl, Methoxy, Ethoxy, tert-Butoxy, Cyclopropyl; Trifluormethyl, Trifluormethoxy oder 4-(Difluormethyl)-2-methyl-1,3-thiazol-2-yl.
- R⁷: steht ganz besonders bevorzugt für Wasserstoff oder 4-(Difluormethyl)-2-methyl-1,3-thiazol-2-yl.
- R⁸ und R⁹: stehen unabhängig voneinander bevorzugt für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁸ und R⁹: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹³ enthalten kann.
- R⁸ und R⁹: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl,; Trifluormethyl, Trichlormethyl, Trifluorethyl, Trifluormethoxymethyl.
- R⁸ und R⁹: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹³ substituiert sein kann.
- R¹⁰ und: R¹¹ stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor, Chlor- und/oder Bromatomen.
- R¹⁰ und R¹¹: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹³ enthalten kann.
- R¹⁰ und R¹¹: stehen unabhängig voneinander besonders bevorzugt für Wasserstoff, Methyl Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R¹⁰ und R¹¹: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹³ substituiert sein kann.
- R¹²: steht bevorzugt für Wasserstoff oder Methyl.
- R¹²: steht besonders bevorzugt für Wasserstoff.
- R¹³: steht bevorzugt für Wasserstoff oder C₁-C₄-Alkyl.
- R¹³: steht besonders bevorzugt für Wasserstoff, Methyl, Ethyl, n- oder iso-Propyl, n-, iso-, sec- oder tert-Butyl.

Weiterhin bevorzugt sind Thiazol-(bi)cycloalkyl-carboxanilide der Formel (I), in welcher R¹ für Wasserstoff steht.

Weiterhin bevorzugt sind Thiazol-(bi)cycloalkyl-carboxanilide der Formel (I), in welcher R¹ für Methyl steht.

Weiterhin bevorzugt sind Thiazol-(bi)cycloalkyl-carboxanilide der Formel (I), in welcher R¹ für -CHO steht.

Weiterhin bevorzugt sind Thiazol-(bi)cycloalkyl-carboxanilide der Formel (I), in welcher R¹ für Methylcarbonyl (Acetyl) steht.

Weiterhin bevorzugt sind Thiazol-(bi)cycloalkyl-carboxanilide der Formel (I), in welcher Q für Q-1 und R² für Bicyclo[2.2.1]heptyl (Norbornanyl) steht.

Weiterhin bevorzugt sind Thiazol-(bi)cycloalkyl-carboxanilide der Formel (I), in welcher Q für Q-1 und m für 0 steht.

Weiterhin bevorzugt sind Thiazol-(bi)cycloalkyl-carboxanilide der Formel (I), in welcher Q für Q-1, m für 1 und R³ für Fluor oder Methyl steht.

Außerdem bevorzugt sind Thiazol-(bi)cycloalkyl-carboxanilide der Formel (I-a) 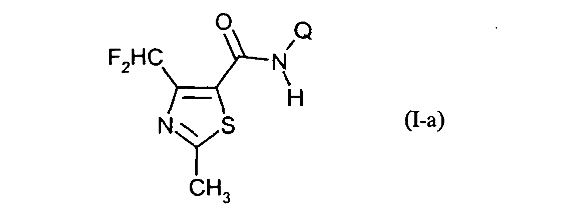 in welcher
- Q: die oben angegebenen Bedeutungen hat.

Außerdem bevorzugt sind Thiazol-(bi)cycloalkyl-carboxanilide der Formel (I-b) 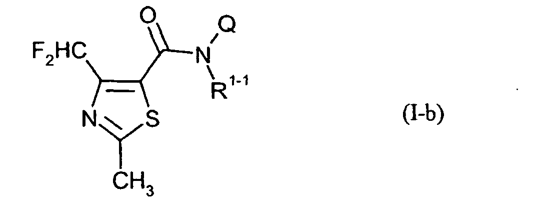 in welcher
- Q: die oben angegebenen Bedeutungen hat,
- R¹⁻¹: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cyaoalkyl; C₁-C₆-Halogenalkyl, C₁-C₄-Halogenalkylsulfanyl, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatome; -COR⁷, -CONR⁸R⁹ oder -CH₂NR¹⁰R¹¹ steht,
- R⁷, R⁸,: R⁹, R¹⁰ und R¹¹ die oben angegebenen Bedeutungen haben.
- R¹⁻¹: steht bevorzugt für C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylsulfanyl, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen; -COR⁷, -CONR⁸R⁹ oder -CH₂NR¹⁰R¹¹.
- R¹⁻¹: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n-oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl,
- R¹⁻¹: Trifluormethyl, Trichlormethyl, Trifluorethyl, Difluormethylsulfanyl, Difluorchlormethylsulfanyl, Trifluormethylsulfanyl, Trifluormethylsulfinyl, Trifluormethylsulfonyl, Trifluormethoxymethyl; -COR⁷, -CONR⁸R⁹ oder -CH₂NR¹⁰R¹¹. steht ganz besonders bevorzugt für Methyl oder -COR⁷.

Außerdem bevorzugt sind Thiazol-(bi)cycloalkyl-carboxanilide der Formel (I-c) 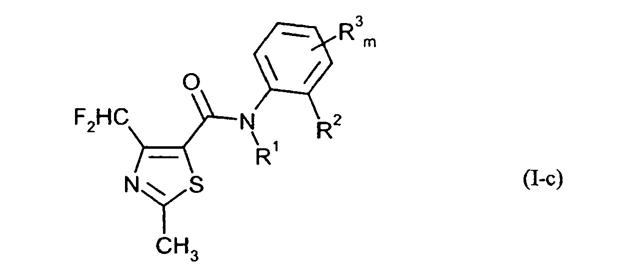 in welcher
- R¹, R² und R³: die oben angegebenen Bedeutungen haben.

Außerdem bevorzugt sind Thiazol-(bi)cycloalkyl-carboxanilide der Formel (I-d) 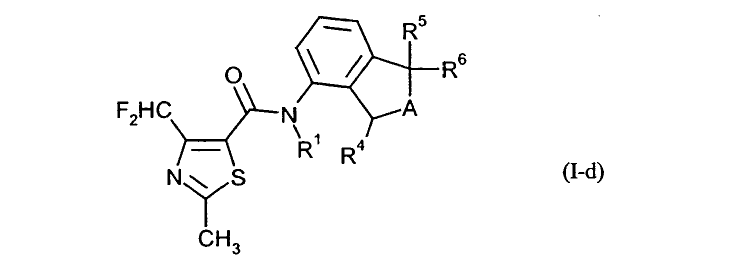 in welcher
A, R⁴, R⁵ und R⁶ die oben angegebenen Bedeutungen haben.

Auf die Verbindungen der Formeln (I-a), (I-b), (I-c) und (I-d) sind die bevorzugten, besonders bevorzugten und ganz besonders bevorzugten Definitionen der jeweiligen Reste R¹ bis R¹³, Q, m und A entsprechend anzuwenden.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenalkyl, sind einfach oder mehrfach Halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Verwendet man 4-(Difluormethyl)-2-methyl-1,3-thiazol-5-carbonylchlorid und 2-Bicyclo[2.2.1]hept-2-ylanilin als Ausgangsstoffe im ersten Schritt und zusätzlich Acetylchlorid als Ausgangsstoff im zweiten Schritt, so kann der Verlauf des erfmdungsgemäßen Verfahrens A) durch das folgende Formelschema veranschaulicht werden.

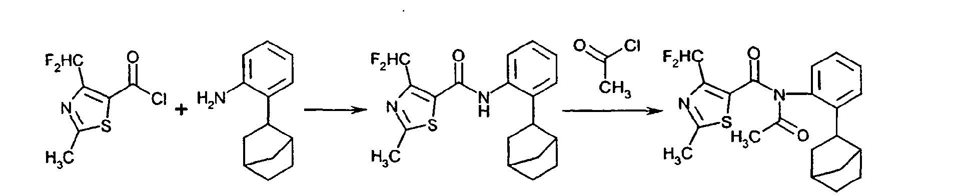

### Erläuterung der Verfahren und Zwischenprodukte

Die bei der Durchführung des erfindungsgemäßen Verfahrens A) im ersten Schritt als Ausgangsstoffe benötigten Carbonsäure-Derivate sind durch die Formel (II) allgemein definiert. In dieser Formel steht G bevorzugt für Chlor, Brom, Hydroxy, Methoxy oder Ethoxy, besonders bevorzugt für Chlor, Hydroxy oder Methoxy, ganz besonders bevorzugt für Chlor.

Die Carbonsäure-Derivate der Formel (II) sind bekannt oder lassen sich nach bekannten Verfahren herstellen (vgl. EP-A 0 545 099 und EP-A 0 589 313).

Die bei der Durchführung des erfindungsgemäßen Verfahrens A) als Reaktionskomponenten im ersten Schritt benötigten Anilin-Derivate sind durch die Formel (III) allgemein definiert. In dieser Formel hat Q vorzugsweise eine der Bedeutungen Q-1 und Q-2.

Die Anilin-Derivate der Formel (III) sind bekannt und/oder lassen sich teilweise nach bekannten Methoden herstellen (vgl. EP-A 0 545 099 und EP-A 0 589 301 im Falle von Q-1, EP-A 0 654 464, EP-A 0 315 502 und EP-A 0 280 275 im Falle von Q-2).

Die bei der Durchführung des erfindungsgemäßen Verfahrens A) als Reaktionskomponenten im zweiten Schritt eingesetzten Verbindungen der Formel (I-a) sind eine Untergruppe der erfindungsgemäßen Thiazol-(bi)cycloalkyl-carboxanilide der Formel (I) und somit ebenfalls Gegenstand dieser Anmeldung.
Die bei der Durchführung des erfindungsgemäßen Verfahrens A) als Reaktionskomponenten im zweiten Schritt benötigten Halogenide sind durch die Formel (IV) allgemein definiert. In dieser Formel hat Q vorzugsweise eine der Bedeutungen Q-1 und Q-2. R¹⁻¹ steht bevorzugt, besonders bevorzugt bzw. ganz besonders bevorzugt für diejenigen Bedeutungen, die bereits im Zusammenhang mit der Beschreibung der erfindungsgemäßen Stoffe der Formel (I-b) für diese Reste als bevorzugt, besonders bevorzugt bzw. ganz besonders bevorzugt angegeben wurden. X steht bevorzugt für Chlor oder Brom.

Halogenide der Formel (III) sind bekannte Synthesechemikalien.

Als Säurebindemittel kommen bei der Durchführung des ersten Schrittes des erfindungsgemäßen Verfahrens A) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert.

Als Verdünnungsmittel kommen bei der Durchführung des ersten Schrittes des erfindungsgemäßen Verfahrens A) jeweils alle üblichen inerten, organischen Solventien infrage. Vorzugsweise verwendbar sind gegebenenfalls Halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan.

Die Reaktionstemperaturen können bei der Durchführung des ersten Schrittes des erfindungsgemäßen Verfahrens A) jeweils in einem größeren Bereich variiert werden. Im allgemeinen arbeitet man bei Temperaturen zwischen 0°C und 140°C, vorzugsweise zwischen 10°C und 120°C.

Bei der Durchführung des ersten Schrittes des erfindungsgemäßen Verfahrens A) arbeitet man im allgemeinen jeweils unter Atmosphärendruck. Es ist aber auch möglich, jeweils unter erhöhtem oder vermindertem Druck zu arbeiten.

Bei der Durchführung des ersten Schrittes des erfindungsgemäßen Verfahrens A) setzt man auf 1 Mol an Carbonsäure-Derivat der Formel (II) im allgemeinen 1 Mol oder auch einen Überschuss an Anilin-Derivat der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Als Verdünnungsmittel zur Durchführung des zweiten Schrittes des erfindungsgemäßen Verfahrens A) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Der zweite Schritt des erfindungsgemäßen Verfahrens A) wird in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des zweiten Schrittes des erfindungsgemäßen Verfahrens A) in einem größeren Bereich variiert werden. Im allgemeinen arbeitet man bei Temperaturen von 0°C bis 150°C, vorzugsweise bei Temperaturen von 20°C bis 110°C.

Bei der Durchführung des zweiten Schrittes des erfindungsgemäßen Verfahrens A) arbeitet man im allgemeinen jeweils unter Atmosphärendruck. Es ist aber auch möglich, jeweils unter erhöhtem oder vermindertem Druck zu arbeiten.

Zur Durchführung des zweiten Schrittes des erfindungsgemäßen Verfahrens (A) zur Herstellung der Verbindungen der Formel (I) setzt man pro Mol des Thiazolylbiphenylamids der Formel (II) im allgemeinen 0,2 bis 5 Mol, vorzugsweise 0,5 bis 2 Mol an Halogenid der Formel (III) ein.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Altemaria, wie Altemaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspeklrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

### Als Mischpartner kommen zum Beispiel folgende Verbindungen in Frage:

### Fungizide:

### 2-Phenylphenol; 8-Hydroxychinolinsulfat;

Acibenzolar-S-methyl; Aldimorph; Amidoflumet; Ampropylfos; Ampropylfos-potassium; Andoprim; Anilazine; Azaconazole; Azoxystrobin;
Benalaxyl; Benodanil; Benomyl; Benthiavalicarb-isopropyl; Benzamacril; Benzamacrilisobutyl; Bilanafos; Binapacryl; Biphenyl; Bitertanol; Blasticidin-S; Bromuconazole; Bupirimate; Buthiobate; Butylamin;
Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram;
Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon;
Edifenphos; Epoxiconazole; Ethaboxam; Ethirimol; Etridiazole;
Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox;
Guazatine; Hexachlorobenzene; Hexaconazole; Hymexazol;
Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl;
Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin;
Natamycin; Nicobifen; Nitrothal-isopropyl; Noviflumuron; Nuarimol;
Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur;
Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole;
Uniconazole; Validamycin A; Vinelozolin; Zineb; Ziram; Zoxamide;
(2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat;Monokaliumcarbonat; N-(6-Methoxy-3-pyridinyl)-cyclopropancarboxamid;
N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxinecopper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin, Japonilure,
Kadethrin, Kernpolyederviren, Kinoprene, Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602; OK-9701, OK-9802, Omethoate, Oxamyl, Oxydemeton-methyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphosmethyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos, Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027, YI-5201, YI-5301, YI-5302, XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze ( z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Namatoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden wieterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucoton^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

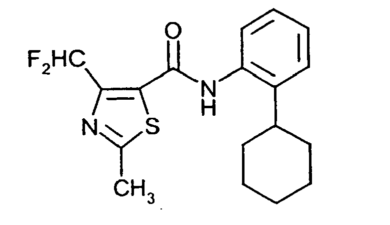

Zu einer Suspension von Kaliumcarbonat (138 mg) in Acetonitril (30 ml) werden 2-Cyclohexylanilin (175 mg, 1 mmol) und 4-(Difluoromethyl)-2-methyl-1,3-thiazole-5-carbonylchlorid (212 mg, 1.2 mmol) getropft. Das Reaktionsgemisch wird für 10 Stunden gerührt.
Zur Aufarbeitung wird das Reaktionsgemisch mit Wasser (30 ml) versetzt und anschließend mit Essigsäureethylester extrahiert. Die organische Phase wird mit Magnesiumsulfat getrocknet, filtriert und eingeengt. Der Rückstand wird an Kieselgel (Gradient Cyclohexan/Essigsäureethylester 100:0 → 20:80) gereinigt.

Man erhält 210 mg (57%) an N-(2-Cyclohexylphenyl)-4-(difluormethyl)-2-methyl-1,3-thiazol-5-carboxamid mit dem logP (pH 2.3) = 3.50

### Beispiel 2

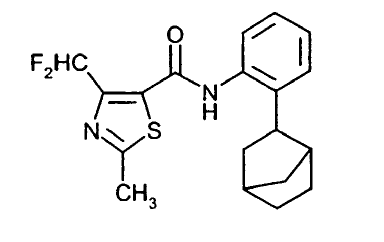

Zu einer Suspension von Kaliumcarbonat (415 mg) in Acetonitril (30 ml) werden 2-Blcyclo[2.2.1]hept-2-ylanilin (562mg, 3 mmol) und 4-(Difluoromethyl)-2-methyl-1,3-thiazole-5-carbonylchlorid (635 mg, 3.6 mmol) getropft. Das Reaktionsgemisch wird für 10 Stunden gerührt.

Zur Aufarbeitung wird das Reaktionsgemisch mit Wasser (30 ml) versetzt und anschließend mit Essigsäureethylester extrahiert. Die organische Phase wird mit Magnesiumsulfat getrocknet, filtriert und eingeengt. Der Rückstand wird an Kieselgel (Gradient Cyclohexan/Essigsäureethylester 100:0 → 20:80) gereinigt.

Man erhielt 150 mg (13 %) an N-(2-Bicyclo[2.2.1]hept-2-ylphenyl)-4-(difluormethyl)-2-methyl-1,3-thiazol-5-carboxamid mit dem logP (pH 2.3) = 3.63

Analog den Beispielen 1 und 2 sowie den allgemeinen Verfahrensbeschreibungen werden auch die in der folgenden Tabelle 1 aufgeführten Verbindungen hergestellt.

**Tabelle 1**

| | | | | | |
|---|---|---|---|---|---|
| 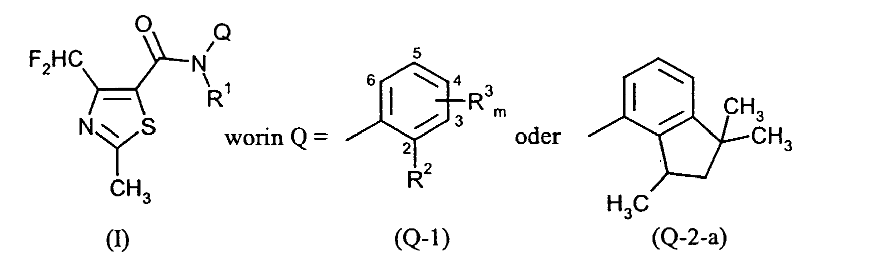 | | | | | |

| **Bsp.** | **Q** | **R¹** | **R²** | **R³ₘ** | **logP (pH2.3)** |
|---|---|---|---|---|---|
| 3 | Q-1 | H | Bicylco[2.2.1]hept-2-yl | 4-CH₃ | 3.92 |
| 4 | Q-1 | H | Bicylco[2.2.1]hept-2-y1 | 5-CH₃ | 4.00 |
| 5 | Q-1 | H | Cycloheptyl | 4-F | 3.84 |
| 6 | Q-1 | H | Cyclopentyl | 4-F | 3.24 |
| 7 | Q-2-a | H | - | - | 3.62 |
| 8 | Q-1 | H | Cyclooctyl | - | 4.06 |
| 9 | Q-1 | H | Cyclooctyl | 4-CH₃ | 4.43 |
| 10 | Q-1 | H | Cyclopentyl | 4,5-(CH₃)₂ | 3.78 |
| 11 | Q-1 | H | Cyclooctyl | 4-F | 4.10 |
| 12 | Q-1 | H | Cyclooctyl | 5-CH₃ | 4.44 |
| 13 | Q-1 | H | Cyclooctyl | 5-F | 4.10 |
| 14 | Q-1 | H | Bicylco[2.2.1]hept-2-yl | 4-Br | 4.27 |
| 15 | Q-1 | H | Bicylco[2.2.1]hept-2-yl | 4,5-(CH₃)₂ | 4.22 |
| 16 | Q-1 | H | Cyclopentyl | - | 3.19 |
| 17 | Q-1 | H | 4-Methylcyclohexyl | - | 3.82 |
| 18 | Q-1 | H | (3*R*)-3-Methylcyclohexyl | - | 3.88 |
| 19 | Q-1 | CH₃ | Bicylco[2.2.1]hept-2-yl | - | 3.96 |
| 20 | Q-1 | CH₃ | Cyclohexyl | - | 3.85 |
| 21 | Q-1 | COCH₃ | Bicylco[2.2.1]hept-2-yl | - | 3.95 |
| 22 | Q-1 | COCH₃ | Cyclohexyl | - | 3.86 |
| 23 | Q-1 | H | 2-Cyclohepten-1-yl | 4-F | 3.68 |
| 24 | Q-1 | H | Cycloheptyl | - | 3.81 |

Die Bestimmung der in den Herstellungsbeispielen angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

Eluenten für die Bestimmung im sauren Bereich: 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Sphaerotheca-Test (Gurke) / protektiv

| | | |
|---|---|---|
| Lösungsmittel: | 24,5 | Gewichtsteile Aceton |
| | 24,5 | Gewichtsteile N,N-Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. Nach Antrocknen des Spritzbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A**

| **Sphaerotheca-Test (Gurke) / protektiv** | | | |
|---|---|---|---|
| Wirkstoff | | Aufwandmenge an Wirkstoff in g/ha | Wirkungsgrad in % |
| 6 | 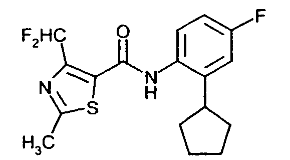 | 100 | 88 |
| 8 | 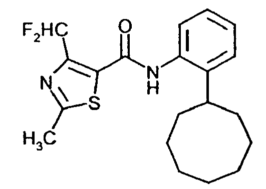 | 100 | 93 |
| 14 | 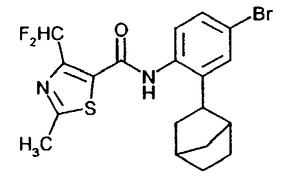 | 100 | 100 |
| 18 | 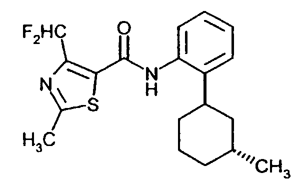 | 100 | 100 |
| 19 | 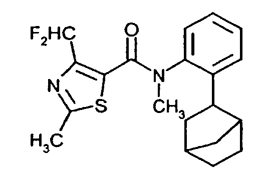 | 100 | 98 |
| 20 | 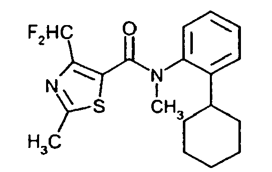 | 100 | 100 |
| 21 | 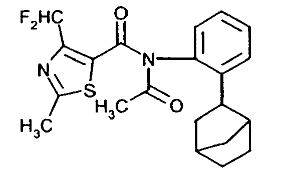 | 100 | 98 |
| 22 | 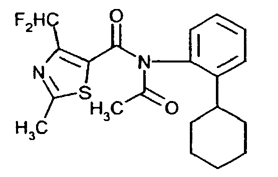 | 100 | 96 |
| 2 | 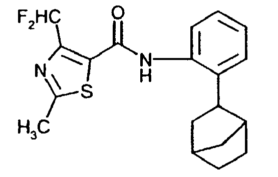 | 100 | 100 |
| 1 | 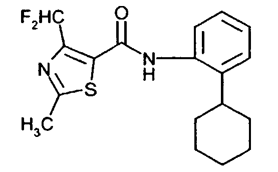 | 100 | 89 |

### Beispiel B

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1,0 | Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle B**

| **Venturia - Test (Apfel) / protektiv** | | | |
|---|---|---|---|
| Wirkstoff | | Aufwandmenge an Wirkstoff in g/ha | Wirkungsgrad in % |
| 6 | 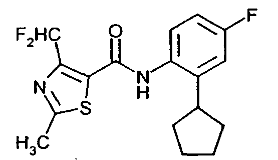 | 100 | 99 |
| 7 | 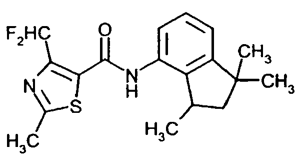 | 100 | 100 |
| 8 | 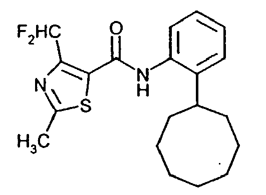 | 100 | 100 |
| 14 | 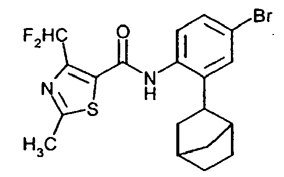 | 100 | 100 |
| 16 | 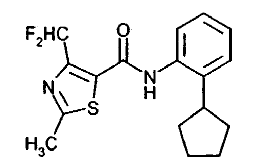 | 100 | 99 |
| 18 | 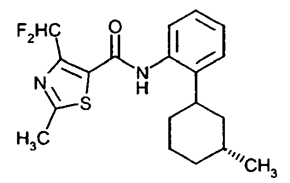 | 100 | 100 |
| 21 | 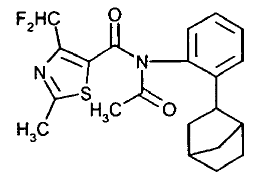 | 100 | 96 |
| 2 | 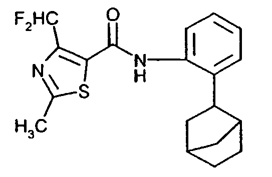 | 100 | 100 |
| 1 | 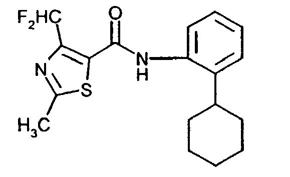 | 100 | 100 |

### Beispiel C

### Puccinia-Test (Weizen) / protektiv

| | |
|---|---|
| Lösungsmittel: | 25 Gewichtsteile N,N-Dimethylacetamid |
| Emulgator: | 0,6 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von *Puccinia recondita* besprüht. Die Pflanzen verbleiben 48 Stunden bei 20°C und 100% relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle C**

| **Puccinia - Test (Weizen) / protektiv** | | | |
|---|---|---|---|
| Wirkstoff | | Aufwandmenge an Wirkstoff in g/ha | Wirkungsgrad in % |
| 7 | 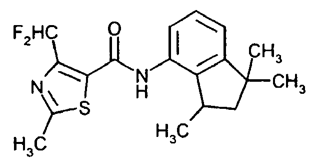 | 500 | 100 |
| 8 | 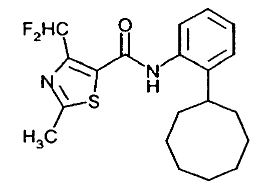 | 500 | 100 |
| 22 | 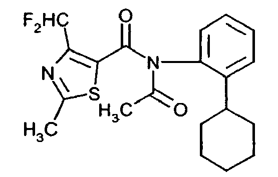 | 500 | 93 |
| 2 | 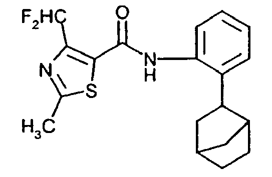 | 500 | 100 |
| 1 | 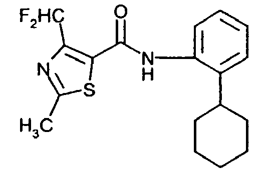 | 500 | 100 |

### Beispiel D

### Alternaria-Test (Tomate) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert. Die Pflanzen stehen dann 24 Stunden bei 100 % relativer Luftfeuchtigkeit und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle D**

| **Alternaria-Test (Tomate) / protektiv** | | | |
|---|---|---|---|
| Wirkstoff | | Aufwandmenge an Wirkstoff in g/ha | Wirkungsgrad in % |
| 7 | 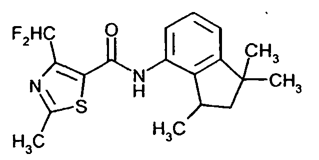 | 750 | 100 |
| 14 | 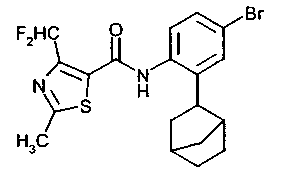 | 750 | 95 |
| 18 | 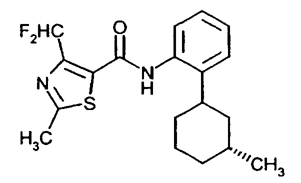 | 750 | 100 |
| 21 | 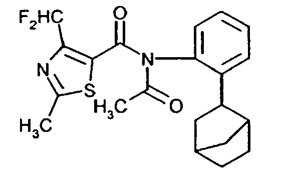 | 750 | 100 |

## Patentansprüche

1. Thiazol-(bi)cycloalkyl-carboxanilide der Formel (I) 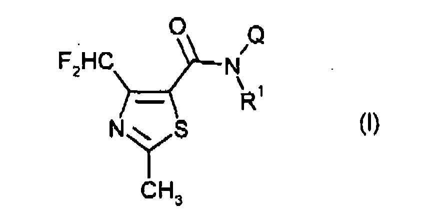 in welcher
Q für eine Gruppe 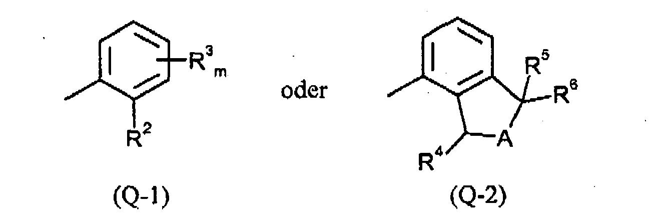 steht,
R¹ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylsulfanyl, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
R² für jeweils gegebenenfalls einfach oder mehrfach, gleich oder verschieden durch Halogen, Cyano, Hydroxy, C₁-C₈-Alkyl, C₁-C₈-Alkoxy, C₁-C₆-Halogenalkyl, C₁-C₆-Halogenalkoxy mit jeweils 1 bis 9 Fluor-, Chlor- und/oder Bromatomen substituiertes C₃-C₁₂-Cycloalkyl, C₃-C₁₂-Cycloalkenyl, C₆-C₁₂-Bicycloalkyl oder C₆-C₁₂-Bicycloalkenyl steht,
R³ für Fluor, Chlor, Brom oder Methyl steht,
m für 0, 1, 2, 3 oder 4 steht,
A für O (Sauerstoff) oder CR¹² steht,
R⁴, R⁵, R⁶ und R¹² unabhängig voneinander für Wasserstoff, Methyl oder Ethyl stehen,
R⁷ für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen oder 4-(Difluormethyl)-2-methyl-1,3-thiazol-2-yl steht,
R⁸ und R⁹ unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R⁸ und R⁹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht beachbarte Heteroatome aus der Reihe Sauerstoff, Schwefel oder NR¹³ enthalten kann,
R¹⁰ und R¹¹ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R¹⁰ und R¹¹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
R¹³ für Wasserstoff oder C₁-C₆-Alkyl steht.

2. Thiazol-(bi)cycloalkyl-carboxanilide der Formel (I) gemäß Anspruch 1, in welcher
Q für eine Gruppe 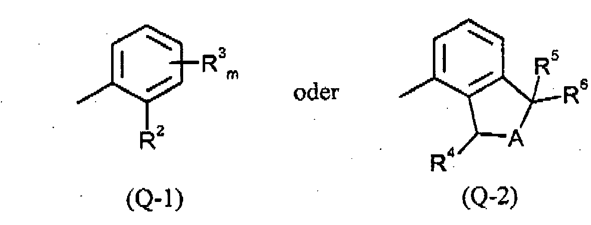 steht,
R¹ für Wasserstoff: C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylsulfanyl, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
R² für jeweils gegebenenfalls einfach bis vierfach, gleich oder verschieden durch Fluor, Chlor, Brom, Cyano, Hydroxy, C₁-C₆-Alkyl, C₁-C₆-Alkoxy, C₁-C₄-Halogenalkyl, C₁-C₄-Halogenalkoxy mit jeweils 1 bis 9 Fluor-, Chlor- und/oder Bromatomen substituiertes C₃-C₁₂-Cycloalkyl substituiertes C₃-C₁₂-Cycloalkyl, C₃-C₁₂-Cycloalkenyl, C₆-C₁₂-Bicycloalkyl oder C₆-C₁₂-Bicycloalkenyl steht,
R³ für Fluor, Brom oder Methyl steht,
m für 0, 1, 2 oder 3 steht,
A für O (Sauerstoff) oder CR¹² steht,
R⁴, R⁵, R⁶ und R¹² unabhängig voneinander für Wasserstoff, Methyl oder Ethyl stehen,
R⁷ für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen oder 4-(Difiuormethyl)-2-methyl-1,3-thiazol-2-yl steht,
R⁸ und R⁹ unabhängig voneinander für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen stehen,
R⁸ und R⁹ außerdem gemeinsam mit dem Stickstoffatom, an das sie gebunden sind, einen gegebenenfalls einfach bis vierfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
R¹⁰ und R¹¹ unabhängig voneinander für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen stehen,
R¹⁰ und R¹¹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R¹³ für Wasserstoff oder C₁-C₄-Alkyl steht.

3. Thiazol-(bi)cycloalkyl-carboxanilide der Formel (I) gemäß Anspruch 1, in welcher
Q für eine Gruppe 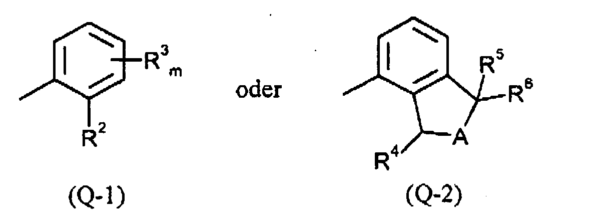 steht,
R¹ für Wasserstoff, Methyl, Ethyl, n- oder iso-Propyl, n-, iso-, sec- oder tert-Butyl, Pentyl oder Hexyl, Methylsulfinyl, Methylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylsulfanyl, Difluorchlormethylsuffanyl, Trifluormethylsulfanyl, Trifluormethylsulfinyl, Trifluormethylsulfonyl, Trifluormethoxymethyl; -COR⁷, -CONR⁸R⁹ oder -CH₂NR¹⁰R¹¹ steht,
R² für jeweils gegebenenfalls einfach bis dreifach, gleich oder verschieden durch Fluor, Chlor, Brom, Cyano, Hydroxy, Methyl, Ethyl, n- oder iso-Propyl, n-, iso-, sec- oder tert-Butyl, Methoxy, Ethoxy, n- oder iso-Propoxy, n-, iso-, sec- oder tert-Butoxy, Trifluormethyl, Difluormethyl, Trichlormethyl, Difluorchlormethyl, Trifluormethoxy, Difluormethoxy, Trichlormethoxy, Difluorchlormethoxy substituiertes C₃-C₁₀-Cycloalkyl, C₃-C₁₀-Cycloalkenyl, C₆-C₁₀-Bicycloalkyl oder C₆-C₁₀-Bicycloalkenyl steht,
R³ für Fluor, Brom oder Methyl steht,
m für 0, 1, 2 oder 3 steht,
A für O (Sauerstoff) oder CR¹² steht,
R⁴ für Methyl oder Ethyl steht,
R⁵ und R⁶ jeweils für Methyl stehen,
R⁷ für Wasserstoff, Methyl, Ethyl, n- oder iso-Propyl, tert-Butyl, Methoxy, Ethoxy, tert-Butoxy, Cyclopropyl; Trifluormethyl, Trifluormethoxy oder 4-(Difluormethyl)-2-methyl-1,3-thiazol-2-yl steht,
R⁸ und R⁹ unabhängig voneinander für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl stehen,
R⁸ und R⁹ außerdem gemeinsam mit dem Stickstoffatom, an das sie gebunden sind, einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin bilden, wobei das Piperazin am zweiten Stickstoffatom durch R¹³ substituiert sein kann,
R¹⁰ und R¹¹ unabhängig voneinander für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl stehen,
R¹⁰ und R¹¹ außerdem gemeinsam mit dem Stickstoffatom, an das sie gebunden sind, einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin bilden, wobei das Piperazin am zweiten Stickstoffatom durch R¹³ substituiert sein kann,
R¹² für Wasserstoff oder Methyl steht,
R¹³ für Wasserstoff, Methyl, Methyl, n- oder iso-Propyl, n-, iso-, sec- oder tert-Butyl steht.

4. Thiazol-(bi)cycloalkyl-carboxanilide der Formel (I-a) 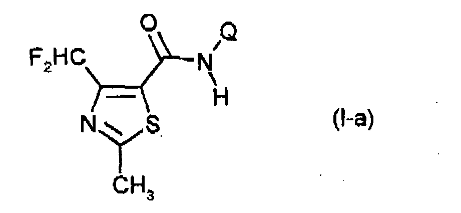 in welcher
Q die in Anspruch 1 angegebenen Bedeutungen hat.

5. Thiazol-(bi)cycloalkyl-carboxanilide der Formel (I-b) 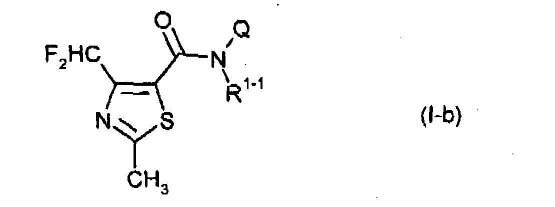 in welcher
Q die in Anspruch 1 angegebenen Bedeutungen hat.
R¹⁻¹ für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Hafogenalkylsulfanyl, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
R⁷, R⁸, R⁹, R¹⁰ und R¹¹ die in Anspruch 1 angegebenen Bedeutungen haben.

6. Thiazol-(bi)cycloalkyl-carboxanilide der Formel (I-c) 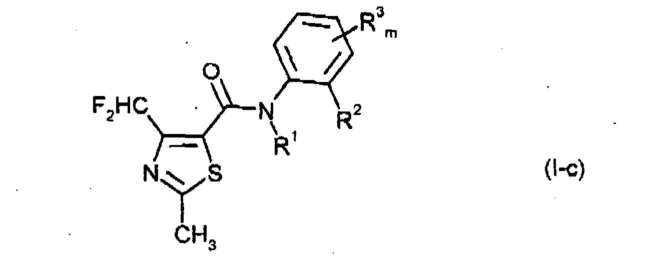 in welcher
R¹, R² und R³ die in Anspruch 1 angegebenen Bedeutungen haben.

7. Thiazol-(bi)cycloalkyl-carboxanilide der Formel (I-d) 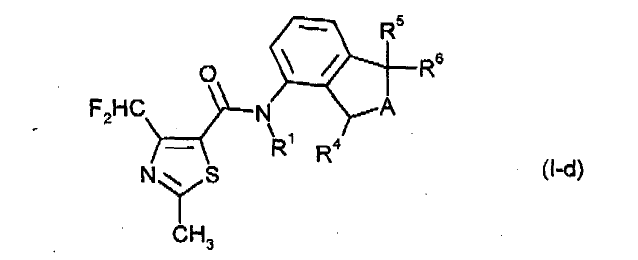 in welcher
A, R⁴, R⁵ und R⁶ die in Anspruch 1 angegebenen Bedeutungen haben.

8. Verfahren zum Herstellen von Thiazol-(bi)cycloalkyl-carboxaniliden der Formel (I) gemäß Anspruch 1, **dadurch gekennzeichnet, dass** man
A) Carbonsäure-Derivate der Formel (II) 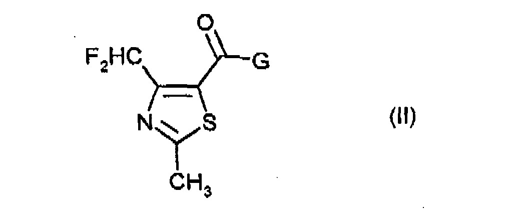
in welcher
G für Halogen, Hydroxy oder C₁-C₆-Alkoxy steht,
in einem ersten Schritt mit Anilin-Derivaten der Formel (III)
H₂N-Q (III)
in welcher
Q die in Anspruch 1 angegebenen Bedeutungen hat,
in Gegenwart eines Säurebindemittels und in Gegenwart eines Verdünnungsmittels umsetzt
und die so erhaltenen Produkte der Formel (I-a) 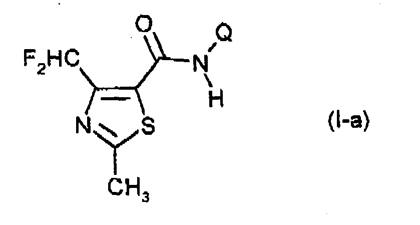 in welcher
Q die in Anspruch 1 angegebenen Bedeutungen hat,
gegebenenfalls in einem zweiten Schritt mit einem Halogenid der Formel (III)
R¹⁻¹-X (IV)
in welcher
R¹⁻¹ für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl. C₁-C₄-Halogenalkylsulfanyl, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
R⁷, R⁸, R⁹, R¹⁰ und R¹¹ die in Anspruch 1 angegebenen Bedeutungen haben und
X für Chlor, Brom oder lod steht,
in Gegenwart einer Base und in Gegenwart eines Verdünnungsmittels umsetzt.

9. Mittel zur Bekämpfung unerwünschter Mikroorganismen, **gekennzeichnet durch** einen Gehalt an mindestens einem Thiazol-(bi)cycloalkyl-carboxanilid der Formel (I) gemäß Anspruch 1 neben Streckmitteln und/oder oberflächenaktiven Stoffen.

10. Verwendung von Thiazol-(bi)cycloalkyl-carboxaniliden der Formel (I) gemäß Anspruch 1 zur Bekämpfung unerwünschter Mikroorganismen, im Pflanzenschutz und im Materialschutz.

11. Verfahren zur Bekämpfung unerwünschter Mikroorganismen im Pflanzenschutz und im Materialschutz, **dadurch gekennzeichnet, dass** man Thiazol-(bi)cycloalkyl-carboxanilide der Formel (I) gemäß Anspruch 1 auf die Mikroorganismen und/oder deren Lebensraum ausbringt.

12. Verfahren zur Herstellung von Mitteln zur Bekämpfung unerwünschter Mikroorganismen im Pflanzenschutz und im Materialschutz, **dadurch gekennzeichnet, dass** man Thiazol-(bi)cycloalkyl-carboxanilide der Formel (I) gemäß Anspruch 1 mit Streckmitteln und/oder oberflächenaktiven Stoffen vermischt.

## Claims

1. Thiazole(bi)cycloalkylcarboxanilides of the formula (I) 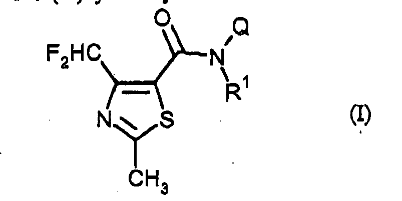 in which
Q represents a group 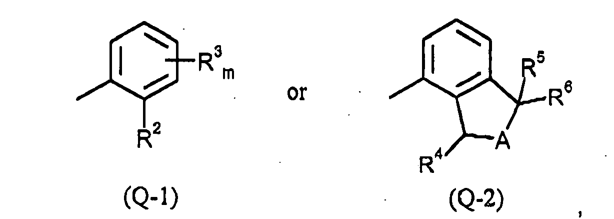
R¹ represents hydrogen, C₁-C₈-alkyl, C₁-C₆-alkylsulfinyl, C₁-C₆-alkylsulfonyl, C₁-C₄-alkoxy-C₁-C₄-alkyl, C₃-C₈-cycloalkyl; C₁-C₆-haloalkyl, C₁-C₄-haloalkylsulfanyl, C₁-C₄-haloalkylsulfinyl, C₁-C₄-haloalkylsulfonyl, halo-C₁-C₄-alkoxy-C₁-C₄-alkyl, C₃-C₈-halocycloalkyl having in each case 1 to 9 fluorine, chlorine and/or bromine atoms; -COR⁷, -CONR⁸R⁹ or -CH₂NR¹⁰R¹¹,
R² represents C₃-C₁₂-cycloalkyl, C₃-C₁₂-cycloalkenyl, C₆-C₁₂-bicycloalkyl or C₆-C₁₂-bicycloalkenyl, each of which is optionally mono- or polysubstituted by identical or different substituents from the group consisting of halogen, cyano, hydroxyl, C₁-C₈-alkyl, C₁-C₈-alkoxy, C₁-C₆-haloalkyl, C₁-C₆-haloalkoxy having in each case 1 to 9 fluorine, chlorine and/or bromine atoms,
R³ represents fluorine, chlorine, bromine or methyl,
m represents 0, 1, 2, 3 or 4,
A represents O (oxygen) or CR¹²,
R⁴, R⁵, R⁶ and R¹² independently of one another represent hydrogen, methyl or ethyl,
R⁷ represents hydrogen, C₁-C₈-alkyl, C₁-C₈-alkoxy, C₁-C₄-alkoxy-C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms or 4-(difluoromethyl)-2-methyl-1,3-thiazol-2-yl,
R⁸ and R⁹ independently of one another represent hydrogen, C₁-C₈-alkyl, C₁-C₄-alkoxy-C₁-C₄-alkyl, C₃-C₈-cycloalkyl; C₁-C₈-haloalkyl, halo-C₁-C₄-alkoxy-C₁-C₄-alkyl, C₃-C₈-halocycloalkyl having in each case 1 to 9 fluorine, chlorine and/or bromine atoms,
R⁸ and R⁹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulfur and NR¹³,
R¹⁰ and R¹¹ independently of one another represent hydrogen, C₁-C₈-alkyl, C₃-C₈-cycloalkyl; C₁-C₈-haloalkyl, C₃-C₈-halocydoalkyl having in each case 1 to 9 fluorine, chlorine and/or bromine atoms,
R¹⁰ and R¹¹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulfur and NR¹³,
R¹³ represents hydrogen or C₁-C₆-alkyl.

2. Thiazole(bi)cycloallcylcaxboxanilides of the formula (I) according to Claim 1 in which
Q represents a group 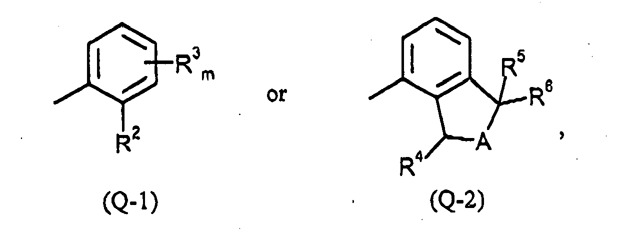
R¹ represents hydrogen; C₁-C₆-alkyl, C₁-C₄-alkylsulfinyl, C₁-C₄-alkylsulfonyl, C₁-C₃-alkoxy-C₁-C₃-alkyl, C₃-C₆-cydoalkyl; C₁-C₄-haloalkyl, C₁-C₄-haloalkylsulfanyl, C₁-C₄-haloalkylsulfinyl, C₁-C₄-haloalkylsulfonyl, halo-C₁-C₃-alkoxy-C₁-C₃-alkyl, C₃-C₆-halocycloalkyl having in each case 1 to 9 fluorine, chlorine and/or bromine atoms; -COR⁷, -CONR⁸R⁹ or -CH₂NR¹⁰R¹¹,
R² represents C₃-C₁₂-cycloalkyl, C₃-C₁₂-cycloalkyl, C₃-C₁₂-cycloalkenyl, C₆-C₁₂-bicycloalkyl or C₆-C₁₂-bicycloalkenyl, each of which is optionally mono- to tetrasubstituted by identical or different substituents from the group consisting of fluorine, chlorine, bromine, cyano, hydroxyl, C₁-C₆-alkyl, C₁-C₆-alkoxy, C₁-C₄-haloalkyl, C₁-C₄-haloalkoxy having in each case 1 to 9 fluorine, chlorine and/or bromine atoms,
R³ represents fluorine, bromine or methyl,
m represents 0, 1, 2 or 3,
A represents O (oxygen) or CR¹²,
R⁴, R⁵, R⁶ and R¹² independently of one another represent hydrogen, methyl or ethyl,
R⁷ represents hydrogen, C₁-C₆-alkyl, C₁-C₄-alkoxy, C₁-C₃-alkoxy-C₁-C₃-alkyl, C₃-C₆-cycloalkyl; C₁-C₄-haloalkyl, C₁-C₄-haloalkoxy, halo-C₁-C₃-alkoxy-C₁-C₃-alkyl, C₃-C₆-halocycloalkyl having in each case 1 to 9 fluorine, chlorine and/or bromine atoms or 4-(difluoromethyl)-2-methyl-1,3-thiazol-2-yl,
R⁸ and R⁹ independently of one another represent hydrogen, C₁-C₆-alkyl, C₁-C₃-alkoxy-C₁-C₃-alkyl, C₃-C₆-cycloalkyl; C₁-C₄-haloalkyl, halo-C₁-C₃-alkoxy-C₁-C₃-alkyl, C₃-C₆-halocycloalkyl having in each case 1 to 9 fluorine, chlorine and/or bromine atoms,
R⁸ and R⁹ furthermore together with the nitrogen atom to which they are attached form a saturated heterocycle which is optionally mono- to tetrasubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulfur and NR¹³,
R¹⁰ and R¹¹ independently of one another represent hydrogen, C₁-C₆-alkyl, C₃-C₆-cycloalkyl; C₁-C₄-haloalkyl, C₃-C₆-halocycloalkyl having in each case 1 to 9 fluorine, chlorine and/or bromine atoms,
R¹⁰ and R¹¹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulfur and NR¹²,
R¹³ represents hydrogen or C₁-C₄-alkyl.

3. Thiazole(bi)cycloalkylcarboxanilides of the formula (I) according to Claim 1 in which
Q represents a group 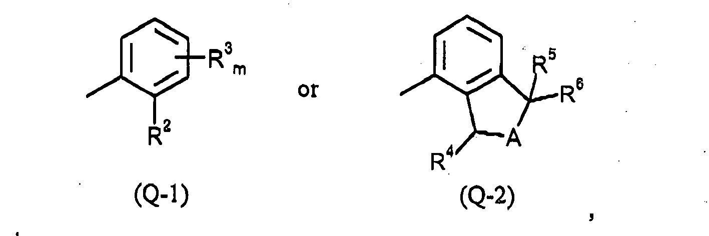
R¹ represents hydrogen, methyl, ethyl, n- or isopropyl, n-, iso-, sec- or tert-butyl, pentyl or hexyl, methylsulfinyl, ethylsulfinyl, n- or isopropylsulfinyl, n-, iso-, sec- or tert-butylsulfinyl, methylsulfonyl, ethylsulfonyl, n- or isopropylsulfonyl, n-, iso-, sec- or tert-butylsulfonyl, methoxymethyl, methoxyethyl, ethoxymethyl, ethoxyethyl, cyclopropyl, cyclopentyl, cyclohexyl, trifluoromethyl, trichloromethyl, trifluoroethyl, difluoromethylsulfanyl, difluorochloromethylsulfanyl, trifluoromethylsulfanyl, trifluoromethylsulfinyl, trifluoromethylsulfonyl, trifluoromethoxymethyl; -COR⁷, -CONR⁸R⁹ or -CH₂NR¹⁰R¹¹,
R² represents C₃-C₁₀-cycloalkyl, C₃-C₁₀-cycloalkenyl, C₆-C₁₀-bicycloalkyl or C₆-C₁₀-bicycloalkenyl, each of which is optionally mono- to trisubstituted by identical or different substituents from the group consisting of fluorine, chlorine, bromine, cyano, hydroxyl, methyl, ethyl, n- or isopropyl, n-, iso-, sec- or tert-butyl, methoxy, ethoxy, n- or isopropoxy, n-, iso-, sec- or tert-butoxy, trifluoromethyl, difluoromethyl, trichloromethyl, difluorochloromethyl, trifluoromethoxy, difluoromethoxy, trichloromethoxy, difluorochloromethoxy,
R³ represents fluorine, bromine or methyl,
m represents 0, 1, 2 or 3,
A represents O (oxygen) or CR¹²,
R⁴ represents methyl or ethyl,
R⁵ and R⁶ each represent methyl,
R⁷ represents hydrogen, methyl, ethyl, n- or isopropyl, tert-butyl, methoxy, ethoxy, tert-butoxy, cyclopropyl; trifluoromethyl, trifluoromethoxy or 4-(difluoromethyl)-2-methyl-1,3-thiazol-2-yl,
R⁸ and R⁹ independently of one another represent hydrogen, methyl, ethyl, n- or isopropyl, n-, iso-, sec- or tert-butyl, methoxymethyl, methoxyethyl, ethoxymethyl, ethoxyethyl, cyclopropyl, cyclopentyl, cyclohexyl; trifluoromethyl, trichloromethyl, trifluoroethyl, trifluoromethoxymethyl,
R⁸ and R⁹ furthermore together with the nitrogen atom to which they are attached form a saturated heterocycle from the group consisting of morpholine, thiomorpholine and piperazine which is optionally mono- to tetrasubstituted by identical or different substituents from the group consisting of fluorine, chlorine, bromine and methyl, where the piperazine may be substituted on the second nitrogen atom by R¹³,
R¹⁰ and R¹¹ independently of one another represent hydrogen, methyl, ethyl, n- or isopropyl, n-, iso-, sec- or tert-butyl, methoxymethyl, methoxyethyl, ethoxymethyl, ethoxyethyl, cyclopropyl, cyclopentyl, cyclohexyl; trifluoromethyl, trichloromethyl, trifluoroethyl, trifluoromethoxymethyl,
R¹⁰ and R¹¹ furthermore together with the nitrogen atom to which they are attached form a saturated heterocycle from the group consisting of morpholine, thiomorpholine and piperazine which is optionally mono- to tetrasubstituted by identical or different substituents from the group consisting of fluorine, chlorine, bromine and methyl, where the piperazine may be substituted on the second nitrogen atom by R¹³,
R¹² represents hydrogen or methyl,
R¹³ represents hydrogen, methyl, ethyl, n- or isopropyl, n-, iso-, sec- or tert-butyl.

4. Thiazole(bi)cycloalkylcarboxanilides of the formula (I-a) 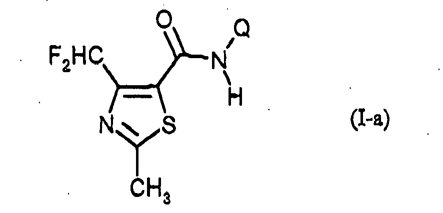 in which
Q is as defined in Claim 1.

5. Thiazole(bi)cycloalkylcarboxanilides of the formula (I-b) 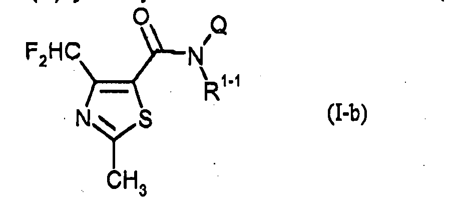 in which
Q is as defined in Claim 1,
R¹⁻¹ represents C₁-C₈-alkyl, C₁-C₆-alkylsulfinyl, C₁-C₆-alkylsulfonyl, C₁-C₄-alkoxy-C₁-C₄-alkyl, C₃-C₈-cycloalkyl; C₁-C₆-haloalkyl, C₁-C₄-haloalkylsulfanyl, C₁-C₄-haloalkylsulfinyl, C₁-C₄-haloalkylsulfonyl, halo-C₁-C₄-alkoxy-C₁-C₄-alkyl, C₃-C₈-halocycloalkyl having in each case 1 to 9 fluorine, chlorine and/or bromine atoms; -COR⁷, -CONR⁸R⁹ or -CH₂NR¹⁰R¹¹,
R⁷, R⁸, R⁹, R¹⁰ and R¹¹ are as defined in Claim 1.

6. Thiazole(bi)cycloalkylcarboxanilides of the formula (I-c) 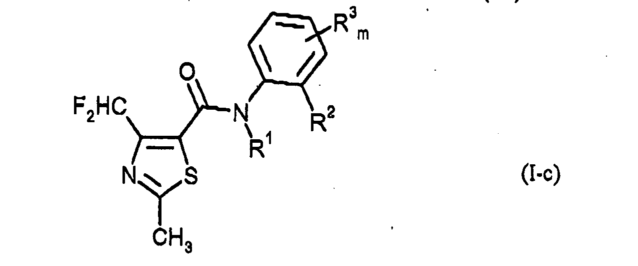 in which
R¹, R² and R³ are as defined in Claim 1.

7. Thiazole(bi)cycloalkylcarboxanilides of the formula (I-d) 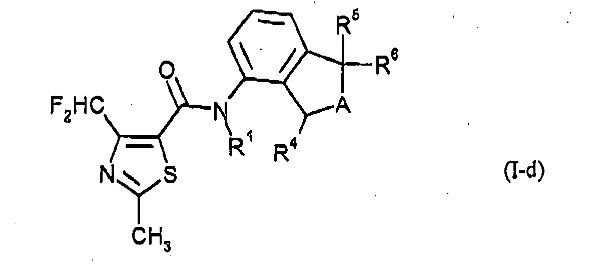 in which
A, R⁴, R⁵ and R⁶ are as define in Claim 1.

8. Process for preparing thiazolo(bi)cycloalkylcarboxanilides of the formula (I) according to Claim 1, **characterized in that**
A) carboxylic acid derivatives of the formula (II) 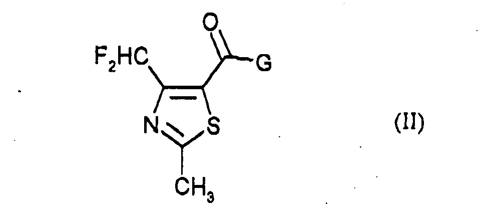
in which
G represents halogen, hydroxyl or C₁-C₆-alkoxy,
are, in a first step, reacted with aniline derivatives of the formula (III)
H₂N-Q (III)
in which
Q is as defined in Claim 1
in the presence of an acid binder and in the presence of a diluent
and the resulting products of the formula (I-a) 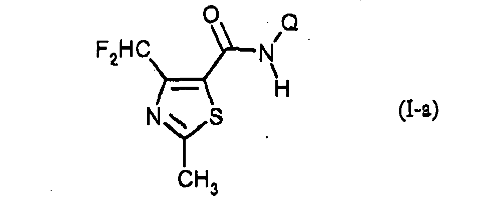 in which
Q is as defined in Claim 1
are, if appropriate, reacted in a second step with a halide of the formula (III)
R¹⁻¹-X (IV)
in which
R¹⁻¹ represents C₁-C₈-alkyl, C₁-C₆-alkylsulfinyl, C₁-C₆-alkylsulfonyl, C₁-C₄-alkoxy-C₁-C₄-alkyl, C₃-C₈-cycloalkyl; C₁-C₆-haloalkyl, C₁-C₄-haloalkylsulfanyl, C₁-C₄-haloalkylsulfinyl, C₁-C₄-haloalkylsulfonyl, halo-C₁-C₄-alkoxy-C₁-C₄-alkyl, C₃-C₈-halocycloalkyl having in each case 1 to 9 fluorine, chlorine and/or bromine atoms; -COR⁷, -CONR⁸R⁹ or -CH₂NR¹⁰R¹¹,
R⁷, R⁸, R⁹, R¹⁰ and R¹¹ are as defined in Claim 1 and
X represents chlorine, bromine or iodine,
in the presence of a base and in the presence of a diluent.

9. Composition for controlling unwanted microorganisms, **characterized in that** it comprises at least one thiazole(bi)cycloalkylcarboxanilide of the formula (I) according to Claim 1, in addition to extenders and/or surfactants.

10. Use of thiazole(bi)cycloalkylcarboxanilides of the formula (I) according to Claim 1 for controlling unwanted microorganisms in crop protection and the protection of materials.

11. Method for controlling unwanted microorganisms in crop protection and the protection of materials, **characterized in that** thiazole(bi)cycloalkylcarboxanilides of the formula (I) according to Claim 1 are applied to the microorganisms and/or their habitat.

12. Process for preparing compositions for controlling unwanted microorganisms in crop protection and the protection of materials, **characterized in that** thiazole(bi)cycloalkylcarboxanilides of the formula (I) according to Claim 1 are mixed with extenders and/or surfactants.

## Revendications

1. Thiazol-(bi)cycloalkyl-carboxanilides de formule (I) 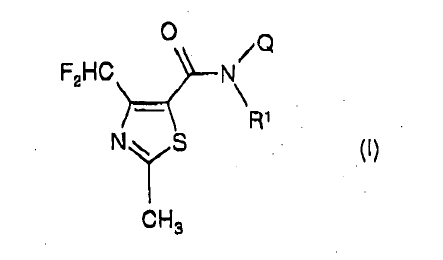 où
Q représente un groupe 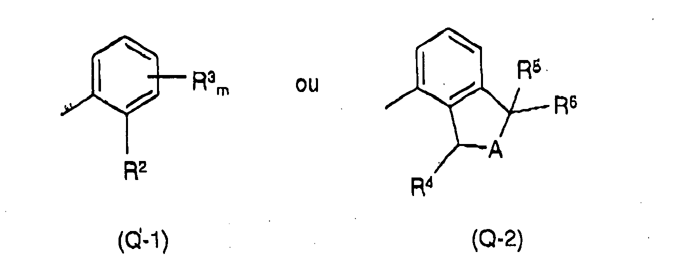
R¹ représente l'hydrogène, C₁-C₈-alkyle, C₁-C₆-alkylsulfinyle, C₁-C₆-alkylsulfonyle, C₁-C₄-alcoxy-C₁-C₄-alkyle, C₃-C₈-cycloalkyle ; C₁-C₆-halogénoalkyle, C₁-C₄-halogéno-alkylsulfanyle, C₁-C₄-halogénoalkylsulfinyle, C₁-C₄-halogénoalkylsulfonyle, halogéno-C₁-C₄-alcoxy-C₁-C₄-alkyle, C₃-C₈-halogénocycloalkyle avec dans chaque cas 1 à 9 atomes de fluor, de chlore et/ou de brome, -COR⁷, -CONR⁸R⁹ ou -CH₂NR¹⁰R¹¹,
R² représente C₃-C₁₂-cycloalkyle, C₃-C₁₂-cycloalcéryle, C₆-C₁₂-bicycloalkyle ou C₆-C₁₂-bicycloalcényle, dans chaque cas éventuellement substitué une ou plusieurs fois, de manière Identique ou différente, par halogène, cyano, hydroxy, C₁-C₈-alkyle, C₁-C₈-alcoxy, C₁-C₆-halogéno-alkyle, C₁-C₆-halogénoalcoxy avec dans chaque cas 1 à 9 atomes de fluor, de chlore et/ou de brome,
R³ représente le fluor, le chlore, le brome ou méthyle,
m représente 0, 1, 2, 3 ou 4,
A représente O (oxygène) ou CR¹²,
R⁴, R⁵, R⁶ et R¹² représentent indépendamment les uns des autres l'hydrogène, méthyle ou éthyle,
R⁷ représente l'hydrogène, C₁-C₈-alkyle, C₁-C₈-alcoxy, C₁-C₄-alcoxy-C₁-C₄-alkyle, C₃-C₈-cycloalkyle ; C₁-C₆-halogénoalkyle, C₁-C₈-halogénoalcoxy, halogéno-C₁-C₄-alcoxy-C₁-C₄-alkyle, C₃-C₈-halogénocycloalkyle avec dans chaque cas 1 à 9 atomes de fluor, de chlore et/ou de brome ou 4-(difluorométhyl)-2-méthyl-1,3-thiazol-2-yle,
R⁸ et R⁹ représentent Indépendamment l'un de l'autre l'hydrogène, C₁-C₈-alkyle, C₁-C₄-alcoxy-C₁-C₄-alkyle, C₃-C₈-cycloalkyle ; C₁-C₈-halogénoalkyle, halogéno-C₁-C₄-alcoxy-C₁-C₄-alkyle, C₃-C₈-halogénocycloalkyle avec dans chaque cas 1 à 9 atomes de Fluor, de chlore et/ou de brome,
R⁸ et R⁹ représentent en outre conjointement avec l'atome d'azote auquel ils sont liés un hétérocycle saturé éventuellement substitué une ou plusieurs fois, de manière identique ou différente, par halogène ou C₁-C₄ alkyle, ayant 5 à 8 atomes cycliques, où l'hétérocycle peut contenir 1 ou 2 autres hétéroatomes non voisins de la série de l'oxygène, du soufre ou NR¹³,
R¹⁰ et R¹¹ représentent indépendamment l'un de l'autre l'hydrogène, C₁-C₈-alkyle, C₃-C₈-cycloalkyle ; C₁-C₈-halogénoalkyle, C₃-C₈-halogénocycloalkyle avec dans chaque cas 1 à 9 atomes de fluor, de chlore et/ou de brome,
R¹⁰ et R¹¹ forment en outre conjointement avec l'atome d'azote auquel ils sont liés un hétérocycle saturé éventuellement substitué une ou plusieurs fois, de manière identique ou différente, par halogène ou C₁-C₄-alkyle avec 5 à 8 atomes cyclique, où l'hétérocycle peut contenir un 1 ou 2 autres hétéroatomes non voisins de la série de l'oxygène, du soufre ou NR¹³,
R¹³ représente l'hydrogène ou C₁-C₆-alkyle.

2. Thiazol-(bi)cycloalkyl-carboxanilides de formule (I) selon la revendication 1 où
Q représente un groupe 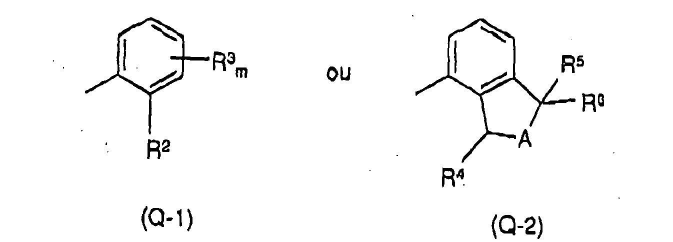
R¹ représente l'hydrogène, C₁-C₆-alkyle, C₁-C₄-alkylsulfinyle, C₁-C₄-alkylsulfonye, C₁-C₃-alcoxy-C₁-C₃-alkyle, C₃-C₆-cycloalkyle ; C₁-C₄-halogénoalkyle, C₁-C₄-halogéno-alkylsulfanyle, C₁-C₄-halogénoalkylsulflnyle, C₁-C₄-halogénoalkylsulfonyle, halogéno-C₁-C₃-alcoxy-C₁-C₃-alkyle, C₃-C₆-halogénocycloalkyle avec dans chaque cas 1 à 9 atomes de fluor, de chlore et/ou de brome, -COR⁷, -CONR⁸R⁹ ou -CH₂NR¹⁰R¹¹,
R² représente C₃-C₁₂-cycloalkyle, C₃-C₁₂-cycloalcényle, C₆-C₁₂-bicycloalkyle ou C₆-C₁₂-bicycloalcényle, dans chaque cas éventuellement substitué une à quatre fois, de manière identique ou différente, par le fluor, le chlore, le brome, cyano, hydroxy, C₁-C₈-alkyle, C₁-C₆-alcoxy, C₁-C₄-halogéno-alkyle, C₁-C₄-halogénoalcoxy avec dans chaque cas 1 à 9 atomes de fluor, de chlore et/ou de brome,
R³ représente le fluor, le brome ou méthyle,
m représente 0, 1, 2 ou 3,
A représente O (oxygène) ou CR¹²,
R⁴, R⁵, R⁶ et R¹² représentent indépendamment les uns des autres l'hydrogène, méthyle ou éthyle,
R⁷ représente l'hydrogène, C₁-C₆-alkyle, C₁-C₄-alcoxy, C₁-C₃-alcoxy-C₁-C₃-alkyle, C₃-C₆-cycloalkyle ; C₁-C₄-halogénoalkyle, C₁-C₄-halogénoalcoxy, halogéno-C₁-C₃-alcoxy-C₁-C₃-alkyle, C₃-C₈-halogénocycloalkyle avec dans chaque cas 1 à 9 atomes de fluor, de chlore et/ou de brome ou 4-(difluorométhyl)-2-méthyl-1,3-thiazol-2-yle,
R⁸ et R⁹ représentent indépendamment l'un de l'autre l'hydrogène, C₁-C₆-alkyle, C₁-C₃-alcoxy-C₁-C₃-alkyle, C₃-C₆-cycloalkyle ; C₁-C₄-halogénoalkyle, halogéno-C₁-C₃-alcoxy-C₁-C₃-alkyle, C₃-C₆-halogénocycloalkyle avec dans chaque cas 1 à 9 atomes de fluor, de chlore et/ou de brome,
R⁸ et R⁹ représentent en outre conjointement avec l'atome d'azote auquel ils sont liés un hétérocycle saturé éventuellement substitué une à quatre fois, de manière identique ou différente, par halogène ou C₁-C₄ alkyle, ayant 5 à 8 atomes cycliques, où l'hétérocycle peut contenir 1 ou 2 autres hétéroatomes non voisins de la série de l'oxygène, du soufre ou NR¹³,
R¹⁰ et R¹¹ représentent indépendamment l'un de l'autre l'hydrogène, C₁-C₆-alkyle, C₃-C₆-cycloalkyle ; C₁-C₄-halogénoalkyle, C₃-C₆-halogénocycloalkyle avec dans chaque cas 1 à 9 atomes de fluor, de chlore et/ou de brome,
R¹⁰ et R¹¹ forment en outre conjointement avec l'atome d'azote auquel ils sont liés un hétérocycle saturé éventuellement substitué une ou plusieurs fois, de manière identique ou différente, par halogène ou C₁-C₄-alkyle avec 5 à 8 atomes cycliques, où l'hétérocycle peut contenir un 1 ou 2 autres hétéroatcmes non voisins de la série de l'oxygène, du soufre ou NR¹²,
R¹³ représente l'hydrogène ou C₁-C₄-alkyle.

3. Thiazol-(bi)cycloalkyl-carboxanilides de formule (I) selon la revendication 1 où
Q représente un groupe 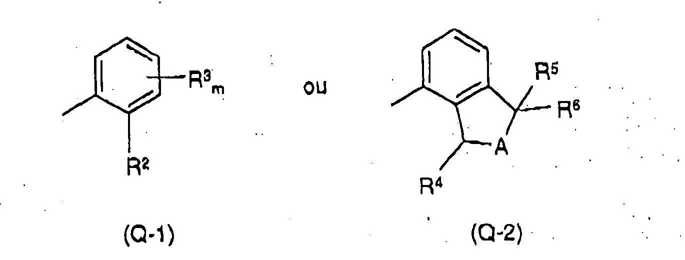
R¹ représente l'hydrogène, méthyle, éthyle, n- ou isopropyle, n-, Iso-, sec- ou tert-butyle, pentyle ou hexyle, méthylsulfinyle, éthylsulfinyle, n- ou iso-propylsulfinyle, n-, iso-, sec- ou tert-butylsulfinyle, méthylsulfonyle, éthylsulfonyle, n- ou iso-propylsulfonyle, n-, iso-, sec- ou tert-butylsulfonyle, méthoxyméthyle, méthoxyéthyle, éthoxyméthyle, éthoxyéthyle, cyclopropyle, cyclopentyle, cyclohexyle, trifluorométhyle, trichlorométhyle, trifluoréthyle, difluorométhylsulfanyle, difluorochlorométhylsulfanyle,
R² trifluorométhylsulfanyle, trifluorométhylsulfinyle, trifluorométhylsulfonyle, trifluorométhoxyméthyle ; -COR⁷, -CONR⁸R⁹ ou -CH₂NR¹⁰R¹¹, représente C₃-C₁₀-cycloalkyle, C₃-C₁₀-cycloalcényle, C₆-C₁₀-bicycloalkyle ou C₆-C₁₀-bicycloalcényle éventuellement substitués 1 à 3 fois, de manière identique ou différente, par le fluor, le chlore, le brome, cyano, hydroxy, méthyle, éthyle, n- ou iso-propyle, n-, iso-, sec- ou tert-butyle, méthoxy, éthoxy, n- ou iso-propoxy, n-, iso-, sec- ou tert-butoxy, trifluorométhyle, difluorométhyle, trichlorométhyle, difluorochlorométhyle, trifluorométhoxy, difluorométhoxy, trichlorométhoxy, difluorochlorométhoxy,
R³ représente le fluor, le brome ou méthyle,
m représente 0, 1, 2 ou 3,
A représente O (oxygène) ou CR¹²,
R⁴ représente méthyle ou éthyle,
R⁵ et R⁶ représentent dans chaque cas méthyle,
R⁷ représente l'hydrogène, méthyle, éthyle, n- ou iso-propyle, tert-butyle, méthoxy, éthoxy, tert-butoxy, cyclopropyle ; trifluorométhyle, trifluorométhoxy ou 4-(difluorométhyl)-2-méthyl-1,3-thiazol-2-yle,
R⁸ et R⁹ représentent indépendamment l'un de l'autre l'hydrogène, méthyle, éthyle, n- ou isopropyle, n-, iso-, sec- ou tert-butyle, méthoxyméthyle, méthoxyéthyle, éthoxyméthyle, éthoxyéthyle, cyclopropyle, cyclopentyle, cycclohexyle, triflorométhyle, trichlorométhyle, trifluoroéthyle, trifluorométhoxyméthyle,
R⁸ et R⁹ forment en outre conjointement avec l'atome d'azote auquel ils sont liés un hétérocycle saturé éventuellement substitué 1 à 4 fois, de manière identique ou différente, par le fluor, le chlore, le brome ou méthyle, de la série de la morpholine, de la thiomorpholine ou de la pipérazine, où la pipérazine peut être substituée par R¹³ sur le second atome d'azote,
R¹⁰ et R¹¹ représentent indépendamment l'un de l'autre l'hydrogène, méthyle, éthyle, n- ou isopropyle, n-, iso-, sec- ou tert-butyle, méthoxyméthyle, méthoxyéthyle, éthoxyméthyle, éthoxyéthyle, cyclopropyle, cyclopentyle, cyclohexyle, trifluorométhyle, trichloro-méthyle, trifluoroéthyle, trifluorométhoxyméthyle,
R¹⁰ et R¹¹ forment en outre conjointement avec l'atome d'azote auquel ils sont liés un hétérocycle saturé éventuellement substitué 1 à 4 fois, de manière Identique ou différente, par le fluor, le chlore, le brome ou méthyle, de la série de la morpholine, de la thiomorpholine ou de la pipérazine, où la pipérazine peut être substituée par R¹³ sur le second atome d'azote,
R¹² représente l'hydrogène ou méthyle,
R¹³ représente l'hydrogène, méthyle, éthyle, n- ou iso-propyle, n-, iso-, sec- ou tert-butyle.

4. Thiazol-(bi)cycloalkyl-carboxanilides de formule (I-a) 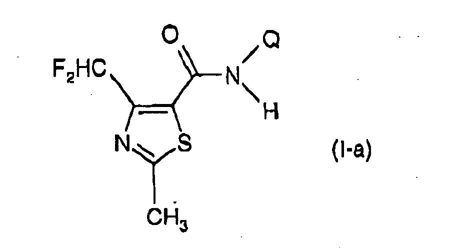 où
Q a les significations indiquées dans la revendication 1.

5. Thiazol-(bi)cycloalkyl-carboxanilides de formule (I-b) 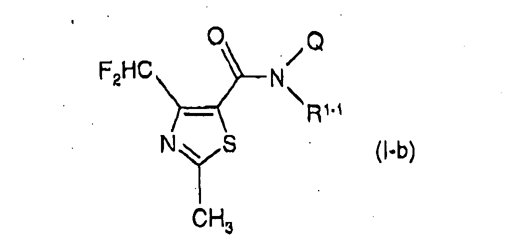 où
Q a les significations indiquées dans la revendication 1,
R¹⁻¹ représente C₁-C₈-alkyle, C₁-C₆-alkylsulfinyle, C₁-C₆-alkylsulfonyle, C₁-C₄-alcoxy-C₁-C₄-alkyle, C₃-C₈-cycloalkyle ; C₁-C₆-halogénoalkyle, C₁-C₄-halogéno-alkylsulfanyle, C₁-C₄-halogénoalkylsulfinyle, C₁-C₄-halogénoalkylsulfonyle, halogéno-C₁-C₄-alcoxy-C₁-C₄-alkyle, C₃-C₈-halogénocycloalkyle avec dans chaque cas 1 à 9 atomes de fluor, de chlore et/ou de brome, -COR⁷, -CONR⁸R⁹ ou -CH₂NR¹⁰R¹¹,
R⁷, R⁸, R⁹, R¹⁰ et R¹¹ ont les significations indiquées dans la revendication 1.

6. Thiazol-(bi)cycloalkyl-carboxanilides de formule (I-c) 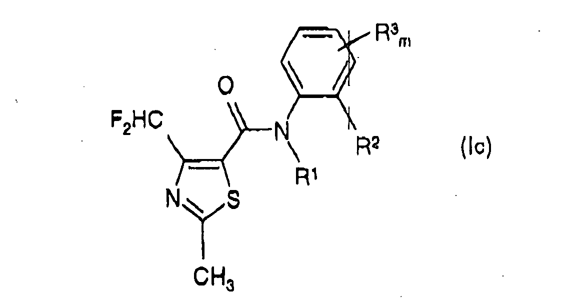 où
R¹, R² et R³ ont les significations indiquée dans la revendication 1.

7. Thiazol-(bi)cycloalkyl-carboxanilides de formule (I-d) 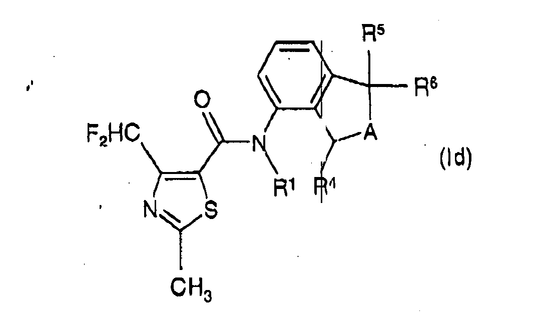 où
A, R¹, R⁵ et R⁶ ont les significations indiquées dans la revendication 1.

8. Procédé pour produire des thiazol-(bi)cycloalkyl-carboxanilides de formule (I) selon la revendication 1, **caractérisé en ce que** l'on fait réagir
A) des dérivés d'acide carboxylique de formule (II) 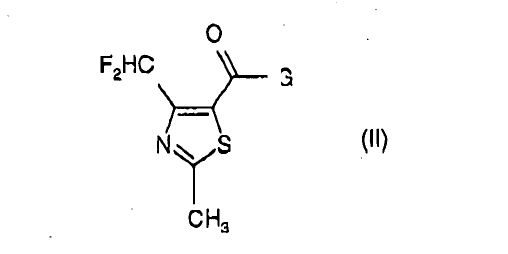
où
G représente halogène, hydroxy ou C₁-C₆-alcoxy,
dans une première étape avec des dérivés de l'aniline de formule (III)
H₂N - Q (III)
où
Q a les significations indiquées dans la revendication 1,
en présence d'un agent liant les acides et en présence d'un diluant
et éventuellement, dans une seconde étape, on fait réagir les produits de formule (I-a) 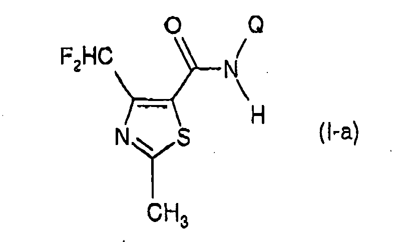 ainsi obtenus, où
Q a les significations indiquées dans la revendication 1,
avec un halogénure de formule (III)
R¹⁻¹ - X (IV)
où
R¹⁻¹ représente C₁-C₈-alkyle, C₁-C₆-alkylsulfinyle, C₁-C₆-alkylsulfonyle, C₁-C₄-alcoxy-C₁-C₄-alkyle, C₃-C₈-cycloalkyle ; C₁-C₆-halogénoalkyle, C₁-C₄-halogéno-alkylsulfanyle, C₁-C₄-halogénoalkylsulfinyle, C₁-C₄-halogénoalkylsulfonyle, halogéno-C₁-C₄-alcoxy-C₁-C₄-alkyle, C₃-C₈-halogénocycloalkyle avec dans chaque cas 1 à 9 atomes de fluor, de chlore et/ou de brome, -COR⁷, -CONR⁸R⁹ ou -CH₂NR¹⁰R¹¹;
R⁷, R⁸, R⁹, R¹⁰ et R¹¹ ont les significations indiquées dans la revendication 1
et
X représente le chlore, le brome ou l'iode,
en présence d'une base et en présence d'un diluant.

9. Agent pour la lutte contre des micro-organismes indésirables, **caractérisé par** une teneur en au moins un thiazol-(bi)cycloalkyl-carboxanilides de formule (I) selon la revendication 1, outre des diluants et/ou des substances tensioactives.

10. Utilisation de thiazol-(bi)cycloalkyl-carboxanilldes de formule (I) selon la revendication 1 pour la lutte contre des micro-organismes indésirables dans la protection des plantes et dans la protection des matériaux.

11. Procédé pour la lutte contre des micro-organismes indésirables dans la protection des plantes et dans la protection des matériaux, **caractérisé en ce que** l'on applique des thiazol-(bi)cycloalkyl-carboxanilides de formule (I) selon la revendication 1 sur les micro-organismes et/ou leur environnement.

12. Procédé pour produire des agents pour la lutte contre des micro-organismes indésirables dans la protection de plantes et dans la protection des matériaux, **caractérisé en ce que** l'on mélange des thiazol-(bi)cycloalkyl-carboxanilides de formule (I) selon la revendication 1 avec des diluants et/ou des substances tensioactives.
